# EUROPEAN PATENT SPECIFICATION

(11) **EP 0 346 343 B1**
(45) Date of publication and mention of the grant of the patent: **03.02.1993**
(21) Application number: 88901147.4
(22) Date of filing: 19.12.1987
(51) Int. Cl.: A61K 9/50, G01N 33/569

(54) **ANTI-VIRAL THERAPEUTIC COMPOSITION**
ANTIVIRALES THERAPEUTIKUM
PREPARATION THERAPEUTIQUE ANTI-VIRALE

(43) Date of publication of application: 20.12.1989
(73) Proprietor: AO-FORSCHUNGSINSTITUT DAVOS, 7270 Davos-Platz (CH)
(72) Inventor: TEPIC, Slobodan, CH-7270 Davos (CH)
(74) Representative: Lusuardi, Werther Giovanni, Dr.
(86) International application number: EP8700806
(87) International publication number: WO8905633

(56) References cited:
- EP-A- 0 017 557
- EP-A- 0 176 429
- EP-A- 0 234 973
- WO-A-81/01153
- WO-A-87/02775
- US-A- 4 652 257
- US-A- 4 663 161

## Description

### Background of the Invention

This invention relates to a therapeutic liposome according to the preamble of claim 1.

Acquired immune deficiency syndrome, or AIDS, is a most threatening pandemic of modern times: Its cause has been identified - it is a human retrovirus, most recently referred to as HIV (Human Immunodeficiency Virus). The virus enters into, resides and replicates within a T4 lymphocyte. Replication of the virus is possible only within an activated lymphocyte. Other cells may also be infected, in particular macrophages, but in contrast to the T4 lymphocyte, these will not be easily killed by the virus. Population of T4 lymphocytes is reduced and disabled by replicating virus, the immune system is compromised, and the patient ultimately dies of some other infection or tumor.

The genetic material of HIV is RNA, which is, together with reverse transcriptase enzyme, encapsulated in the core of the virus consisting of two proteins. In the process of budding out from the host cell the core of the virus gets enveloped in a lipid bi-layer from the outer membrane of the host. The envelope also contains viral glycoproteins - the only functional component of the virus-specific make-up when it is outside a host cell. These envelope glycoproteins are also the only source of antigen of an enveloped virus. Variability in their structure, even within an individual patient, makes the development of a vaccine a difficult task. Most efforts to curb the spread of AIDS and to prevent taking its toll from already infected population are currently directed towards development of an efficient therapy.

A large number of techniques are known in art for treatment against viruses possessing an outer lipid envelope and in particular against AIDS. However, none of the clinically tested therapies to date are satisfactory (AZT, Interferons, Interleukins, Monoclonal antibodies to T4 or to HIV).

As an example it is known from the EP-A-0 176 429 PASTEUR to use a therapeutic composition with an anti-T4 antibody. This can be characterized as a "targeting approach", i.e. not the virus itself is the aim but the cells which are to be affected by the virus. The liposome loaded with the drug goes to the cells which have T4 protein and releases the drug there.

US-A-4 663 161 MANNINO discloses a technique, wherein the membrane proteins of viral origin are integrated into a lipid bilayer around an aqueous space. The liposome compositions and methods of MANNINO do not involve an association of a liposome and a protein which is capable of interacting with the envelope protein of the virus sought to be treated and wherein the liposome engulfs the viral core.

EP-A-0 017 557 MERCK discloses a technique wherein viral or bacterial protein is attached to the liposome, but not to the natural receptor for the bacterial protein.

US-A-4 652 257 CHANG discloses the use of magnetic particles in lipid capsules.

### Summary of the Invention

The invention as claimed is intended to remedy these drawbacks and to provide an efficient therapeutic composition for treating viral diseases, in particular of AIDS and any other infection with the agent's mechanism similar to that of HIV.

It solves the problem of how to design "decoys" for the virus to enter, whereby the virus is stripped of its envelope. A number of steps may be taken following entrapment of the virus, but in no way will it be allowed to return to the state it is in following budding from a host, e.g. a T4 lymphocyte cell.

It is well established that HIV primarily enters cells with the T4 protein in their outer membrane. T4 is abundant in the membrane of the T4 lymphocyte, but is also found in monocytes, macrophages and perhaps, in small numbers, in many other cells. It is probably through the interaction of the viral envelope glycoprotein and the T4 protein that the cell's membrane opens, fusing with the viral envelope, while the viral core enters the cell.
This process is imitated in the method according to the invention by an "artificial cell" - a liposome with T4 molecules, or an equivalent thereof - integrated into the liposome's membrane. Suitable liposomes are those currently used for drug delivery systems - most lately as targeted ones with monoclonal antibodies in their membranes. Their preparation is described e.g. in "Liposome Technology" Vol. I to III edited by Gregory Gregoriadis, published by CRC Press Inc., Boca Raton, Florida (1984).
T4 protein may be purified from the natural cultured cells with the aid of the anti-T4 antibody, or synthetic. Methods for obtaining T4 protein from a culture of T4 lymphocytes are described by C. TERHORST et al. in science (1980), 209, 520-521 and by E.L.REINHERZ et al. in PROC.NATL.ACAD.SCI.USA (1979), Vol. 76, 4061-4065.

Methods for anchoring the T4 protein in the liposome's membrane are described in "Liposome Technology" Vol. I to III edited by Gregory Gregoriadis, published by CRC Press Inc., Boca Raton, Florida (1984).

It is known already to use liposomes containing an antiviral agent with the anti-T4 antibodies in the membrane. These liposomes will be attracted to the T4 carrying cells and release the antiviral agent.
If, however, according to the invention, the T4 protein itself (instead of the anti-T4 antibody) or a part of it responsible for the interaction with the HIV, or an equivalent is incorporated in the membrane, the liposome will become a "decoy" and collect the virus. On entry the virus leaves its envelope behind in the decoy membrane. Virus-collecting efficacy of such decoy liposomes depends on the number of T4 molecules they carry. Size of the decoy plays a certain role, but is not a limiting factor. Surface-to-volume ratio of the virus is higher than that of the decoy so the viral membrane incorporated into liposome's will make for the volume of enclosed core. With increasing number of engulfed virus the membrane will tend to slack out. Liposome diameter is preferably in the range of one to ten µm (microns). In vivo liposomes compete for the virus against the natural T4-carrying cells. Low antigenicity of empty decoys allows for manifold increase of T4 targets and thus reduces the infection rate of natural T4-carrying cells.

Once the virus has been collected by liposomes, a number of steps may be taken to render it harmless:
(1) No further action.
   Supposing that any liposome will eventually be degraded in vivo, the viral cores will find themselves exposed to either blood or lymph. The cores will not find a way to envelop themselves either in the process of liposome degradation, or later. Without its envelope, viral core looses most of its infectivity. It may be destroyed, or it may provoke a better targeted immune response. Thus the simplest therapeutic procedure is based on stripping the virus of it's envelope by T4 marked liposome decoys, which then degrade and dump the viral cores.
(2) Break up decoys by external action. Liposomes can be made to contain a magnetizable particle. Once the virus is collected, these particles can be magnetized by a strong magnetic field and then set in rotation by a rotating magnetic field. This breaks the liposome membrane and spills the content - bare viral cores. To avoid aggregation, magnetic particles could be immediately demagnetized by a high frequence alternating field.
(3) Decoys may be loaded with antiviral agents. Suitable antiviral agents are e.g. heteropolyanionic compositions according to French Patent 73.27.536/2.245.474, ribaverine or lincomycine (marketed under the trademark LYNCOCYNE) or clindamycine as described in "Molecular Basis of Antibiotic Action" by Gale et al. (1972), 678, 2. edition by Wiley and Sons.
   The fact that virus is stripped of its envelope on entry into liposome helps - an enzyme that breaks the core protein(s) suffices. Either natural, or forced destruction of liposomes completes the process.
(4) Decoys may be marked with a second, stable antigen (that even the depressed immune system will react to). Basically as above, but the liposome destruction is sought via immune response.
(5) Activate T4 lymphocytes to bring out dormant virus. Activation may be brought about from outside, e.g. by injection of appropriate lymphokines, or by step (4). On one side liposomes are competing for the virus and provoking the immune response against themselves. Activation pushes infected T4 lymphocytes to express virus, dying in the process and spilling out new generation of virus which is picked up by the decoys. This may offer a chance for thorough elimination of the virus from its major host - T4 lymphocyte.

The various features of novelty which characterize the invention are pointed out with particularity in the claims annexed to and forming part of this disclosure. For the better understanding of the invention, its operating advantages and specific objects attained by its use, reference should be had to the accompanying drawings and descriptive matter in which are illustrated and described preferred embodiments of the invention.

### Brief Description of the Drawings

In the drawings:
Fig. 1 is a schematic representation of the internal structure of a Human Deficiency Virus (HIV);
Fig. 2 is a schematic representation of a virus at the moment of attachment to the host cell;
Fig. 3 is a schematic representation of the virus of Fig. 2 whose lipid layer has fused with that of the host cell;
Fig. 4 is a schematic representation of a new viral core budding out of the host cell of Fig. 2;
Fig. 5 shows a decoy according to the invention consisting of a liposome carrying T4 proteins;
Fig. 6 shows virus in different stages of entry into the decoy of Fig. 5.

### Description of the Preferred Embodiments

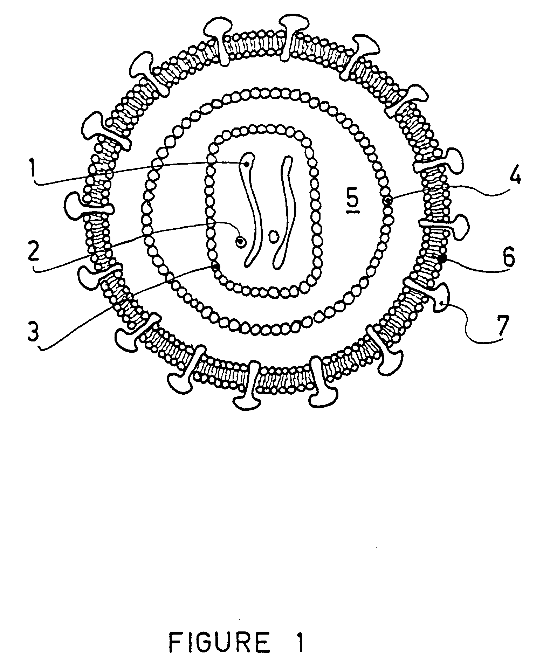

Fig. 1 shows the structure of the Human Immunodeficiency Virus (HIV) as currently understood. The genetic material 1 is in the form of RNA. It is accompanied by reverse transcriptase enzyme 2, which, upon release within the host cell, transcribes ribonucleic acid (RNA) to deoxyribonucleic acid (DNA) to be incorporated into the host own DNA. Two core proteins, 3 and 4 , encapsulate the RNA and the enzyme in the viral core 5. The outer envelope of the virus is a lipid bi-layer 6. Viral envelope glycoproteins 7 are embedded in the lipid bi-layer 6. Diameter of the virus is about one tenth of a micron.

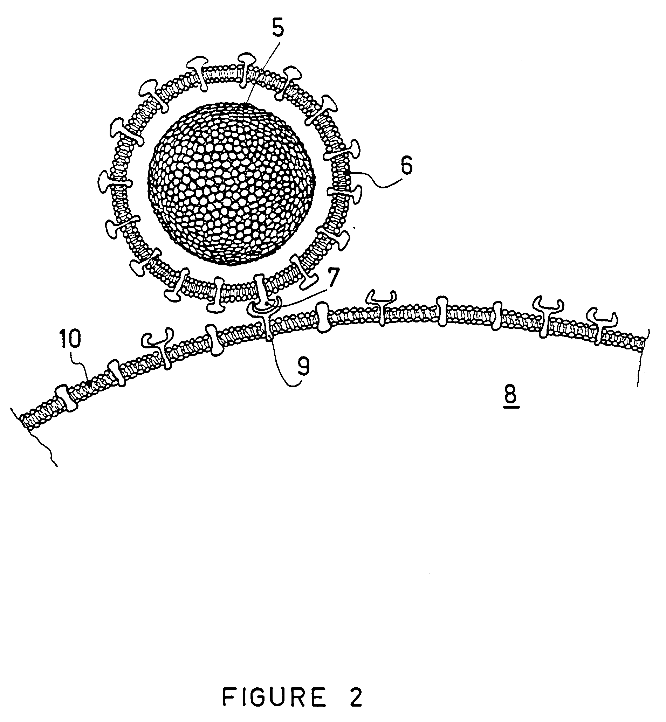

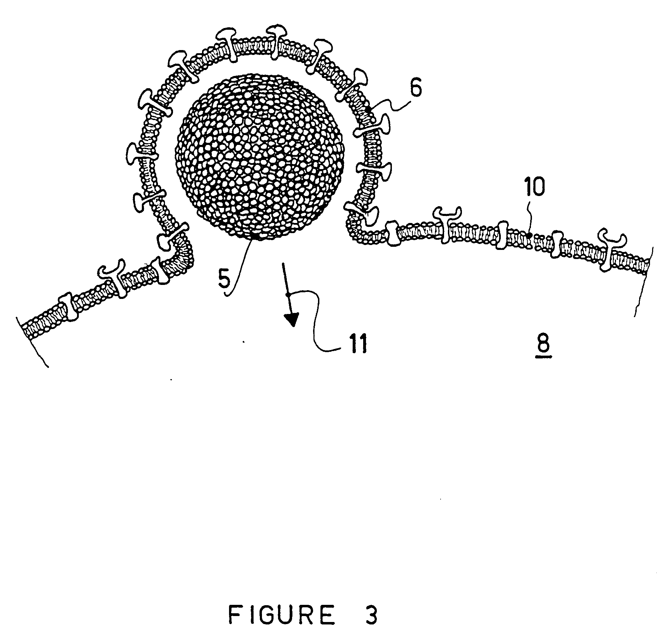

Fig. 2 shows a virus at the moment of attachment to the host cell 8. An envelope glycoprotein 7 binds to a cell membrane protein 9, which to the best current knowledge is the T4 protein. Interaction between proteins 7 and 9 pulls the two lipid layers 6 and 10 together so they can fuse and open a way for the viral core 5 to enter the cell as shown by arrow 11 on Fig. 3. Host cell enzymes proceed to cut the core proteins releasing the RNA and reverse transcriptase.

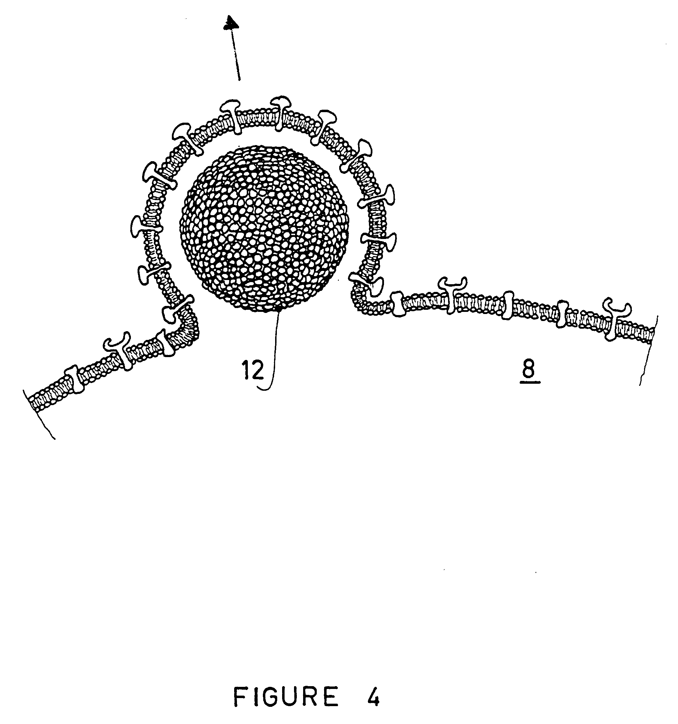

Fig. 4 shows a new viral core 12 budding out from the host cell 8. In the case of the T4 lymphocyte, the cell 8 dies at this time with a burst of new viruses spreading out into circulation.

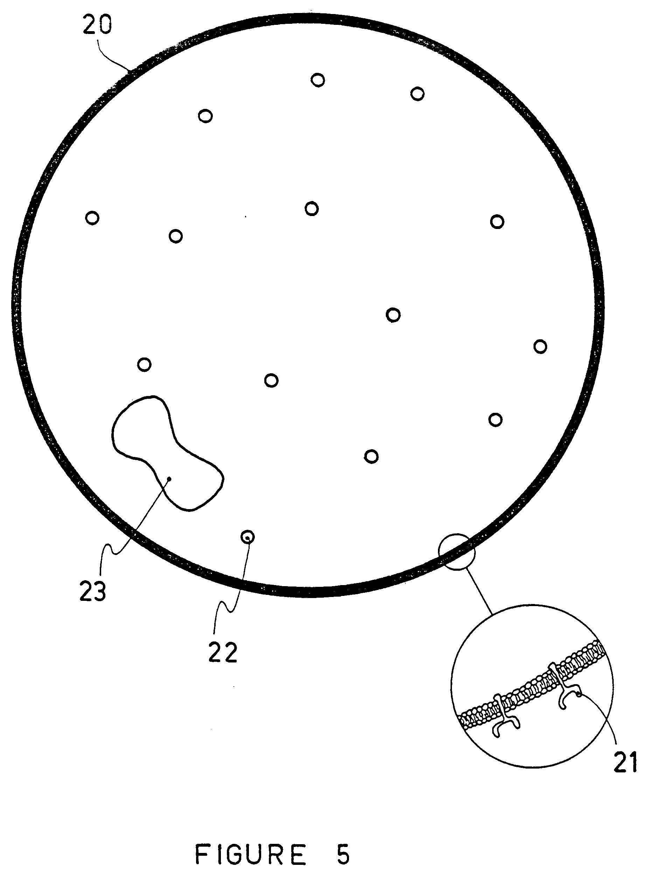

Fig. 5 shows a decoy consisting of a liposome carrying T4 proteins, or an equivalent. Liposome membrane 20 is a lipid bi-layer. It resembles to natural cell membrane. Decoys are not expected to leave circulation and the preferred type of the liposome is a large unilamellar vesicle (LUV) with the diameter between one and ten microns. The T4 protein 21 is incorporated in the decoy membrane 20. It may be complete or only part of it responsible for the entry of the HIV into natural cells. The liposome may additionally be loaded with an antiviral core agent 22. Only the viral core enters the liposome and an enzyme capable of disrupting the viral core proteins will suffice.
The liposome may further contain a magnetizable substance, or particle 23. This allows for external, directed destruction of decoys.

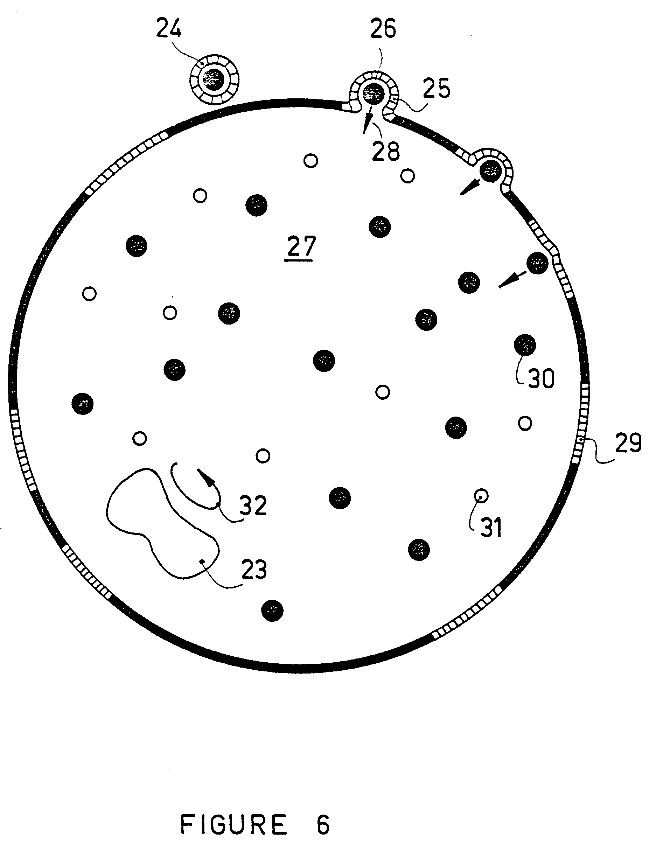

Fig. 6 shows virus in different stages of entry into the decoy. One virus 24 has approached the membrane 20; envelope of another one 25 has fused with the decoy's membrane 20, and its core 26 is entering the liposome's inner space 27 as shown by arrow 28. Membranes 29 of the viral cores 30 already within the liposome are integrated into the liposome membrane 20. Enzyme as a possible anti-viral agent is shown with empty circles 31.
Magnetizable particle 23 may be magnetized and set in rotation by an external magnetic field, as shown by arrow 32. Drag of the surrounding fluid will eventually cause the decoy to rupture, dumping the content into circulation. Particles 23 can be immediately demagnetized to avoid aggregation.

## Claims

1. A therapeutic liposome for use against the class of viruses possessing an outer lipid envelope, characterised by the association of a liposome and the T4 protein whereby the T4 protein (21) is anchored in the liposome's membrane (20), so that upon the viral envelope protein fusion-interaction with the liposome's protein and membrane the liposome engulfs the viral core (26,30).

2. Liposome according to claim 1, characterised in that the liposomes are loaded with an anti-viral agent (31).

3. Liposome according to claim 2, characterised in that the anti-viral agent (31) is a heteropolyanionic composition, ribaverine, lincomycine or clindamycine.

4. Liposome according to claim 3, characterised in that the liposome membrane (20) contains a further antigen besides T4 provoking immune response against the liposome.

5. Liposome according to claim 4, characterised in that it comprises further a magnetizable substance, preferably in particulate form (23), within the liposome.

6. Liposome according to claim 5, characterised in that the liposome is a unilamellar vesicle (LUV).

7. Liposome according to claim 6, characterised in that the diameter of the liposomes is in the range of one to ten µm (microns).

8. Liposome according to claim 1, characterised in that the protein in the liposome's membrane (20) is a fragment of the T4 protein.

## Patentansprüche

1. Ein therapeutisches Liposom zum Einsatz gegen die Klasse von Viren, die eine äußere Lipidhülle besitzen, gekennzeichnet durch die Assoziation eines Liposoms und des T4-Proteins, wodurch das T4-Protein (21) in der Liposommembran (20) verankert wird, so daß bei der Verschmelzungswechselwirkung zwischen dem viralen Hüllenprotein und dem Liposomprotein und Membran das Liposom den Viruskern (26,30) verschlingt.

2. Liposom nach Anspruch 1, dadurch gekennzeichnet, daß die Liposome mit einem Antivirusagens (31) beladen sind.

3. Liposom nach Anspruch 2, dadurch gekennzeichnet, daß das Antivirusagens (31) eine Heteropolyanionenzusammensetzung, Ribaverin, Linkomyzin oder Klindamyzin ist.

4. Liposom nach Anspruch 3, dadurch gekennzeichnet, daß die Liposommembran (20) ein weiteres Antigen neben T4 enthält, das eine Immunabwehr gegen das Liposom provoziert.

5. Liposom nach Anspruch 4, dadurch gekennzeichnet, daß es ferner eine magnetisierbare Substanz, vorzugsweise in Partikelform (23), innerhalb des Liposoms umfaßt.

6. Liposom nach Anspruch 5, dadurch gekennzeichnet, daß das Liposom ein unilamellares Bläschen (LUV) ist.

7. Liposom nach Anspruch 6, dadurch gekennzeichnet, daß der Durchmesser der Liposome im Bereich von ein bis zehn µm (Mikron) ist.

8. Liposom nach Anspruch 1, dadurch gekennzeichnet, daß das Protein in der Liposommembran (20) ein Fragment des T4-Proteins ist.

## Revendications

1. Liposome thérapeutique à utiliser contre la classe des virus possédant une enveloppe lipidique externe, caractérisé par l'association d'un liposome et de la protéine T4, en sorte que la protéine T4 (21) soit ancrée dans la membrane (20) du liposome, si bien que par une interaction-fusion de la protéine de l'enveloppe virale avec la membrane et la proétine du liposome, le liposome engloutit le noyau viral (26, 30).

2. Liposome suivant la revendication 1, caractérisé en ce qu'il est chargé d'un agent antiviral (31).

3. Liposome suivant la revendication 2, caractérisé en ce que l'agent antiviral (31) est une composition hétéropolyanionique, la ribavérine, la lincomycine ou la clindamycine.

4. Liposome suivant la revendication 3, caractérisé en ce que la membrane (20) du liposome contient un autre antigène en plus de T4 provoquant une réponse immunitaire contre le liposome.

5. Liposome suivant la revendication 4, caractérisé en ce qu'il comprend en outre une substance aimantable, de préférence sous forme particulaire (23), dans le liposome.

6. Liposome suivant la revendication 5, caractérisé en ce qu'il est une vésicule unilamellaire (LUV).

7. Liposome suivant la revendication 6, caractérisé en ce que son diamètre varie de 1 à 10 µm (microns).

8. Liposome selon la revendication 1, caractérisé en ce que la protéine dans la membrane (20) du liposome est un fragment de la protéine T4.
